# EUROPEAN PATENT APPLICATION

(11) **EP 2 586 858 A1**
(43) Date of publication of application: **01.05.2013**
(21) Application number: 12405115.2
(22) Date of filing: 26.10.2012
(51) Int. Cl.: C12M 3/00

(54) **Automated cell culture and microscopic observation device.**

(30) Priority: 28.10.2011 CH 17432011
(71) Applicant: Infergen SA, 6900 Massagno (CH)
(72) Inventor: Gianaroli, Luca, 6933 Muzzano (CH); Magli, M. Cristina, 40050 Monte S. Pietro (IT)
(74) Representative: Fabiano, Piero

(57) **Abstract**

Device (100) for culturing, controlling and protecting the development of an embryo in vitro comprising:
- at least one incubator unit (3);
- at least one work unit (2) with microscope; said work unit with microscope comprising at least one microscope (4) and at least one incubator arm (5) extending from said incubator unit (3) to said microscope (4); said incubator arm being in fluid communication with said incubator unit (3);
- at least one input/output unit (1) of the biological material; said at least one input/output unit (1) of the biological material being in fluid communication with the said incubator unit (3).

The incubator unit has at least one handling system for taking a sample of biological material from the input/output unit (1) and bringing it to a work position at the said microscope (4) and/or vice versa.

## Description

### FIELD OF THE INVENTION

The present invention relates to the field of in vitro fecundation (IVF) and more precisely to a device for culturing, controlling and protecting the development of an embryo in vitro.

### PRIOR ART

The infertility levels have dramatically increased in the last decades, to the extent that this condition presently concerns about 20% of the couples. For these couples the use of in vitro fecundation (IVF) turns out to be the only chance for conception.

The present IVF techniques require many interventions of the biologist, included direct manipulation of gametes, zygotes and embryos, as well as their regular monitoring, with a microscope, for controlling the morphology and development during the culturing period. The extracorporeal stage involves the conservation of gametes and embryos in an incubator, so that optimal conditions in terms of temperature, CO2 and humidity can be maintained.

The fecundation of the oocyte and the development of the embryo crucially depend on the ability to keep the above-mentioned parameters (temperature, CO2 and humidity) stable. In particular, the repeated exposure to the outer environment during manipulations can have serious consequences on the correct development of the embryo.

The Applicant has thus faced the problem of making a device for culturing, controlling and protecting the development of an embryo in vitro, which allows the environmental parameters - temperature, pressure, humidity, CO2 - to be controlled and kept substantially constant during the embryo culture period.

The Applicant also faced the problem of making a device for culturing, controlling and protecting the development of an embryo in vitro which is automated, thus reducing to a minimum the exposure to the outer environment and the contact with the operator.

### SUMMARY OF THE INVENTION

In a first aspect thereof, the present invention thus relates to a device for culturing, controlling and protecting the development of an embryo in vitro, comprising:
- at least one incubator unit;
- at least one work unit with microscope; said work unit with microscope comprising at least one microscope and at least one incubator arm extending from said incubator unit to said microscope; said incubator arm being in fluid communication with said incubator unit;
- at least one input/output unit of the biological material; said at least one input/output unit of the biological material being in fluid communication with the said incubator unit;
- said at least one incubator unit comprising at least one handling system for taking a sample of biological material from said input/output unit and bringing it to a work position at the said microscope and/or vice versa.

The present invention, in the above-mentioned aspect, may have at least one of the preferred features hereinafter described.

Preferably, the device for culturing, controlling and protecting the development of an embryo in vitro comprises an atmosphere over-pressurization system comprising a pressurized air circuit comprising:
- at least one pressurized air source;
- at least one filter for the pressurized air; and
- at least one inlet for letting the pressurized air into said incubator unit; said at least one inlet being controlled by at least one valve and by a controller.

Advantageously, the input/output unit of the biological material is contained in a laminar flow hood.

Conveniently, the handling system for taking a sample of biological material from said input/output unit and bringing it to a work position at the said microscope and/or vice versa comprises at least one robot comprising at least one base body, at least one upright element rotatable relative to said base body and at least one crosspiece element translatable along the upright element.

Preferably, the robot further comprises a telescopic arm, movable along said crosspiece element.

Advantageously, the telescopic arm comprises at least two telescopically slidable tubular elements,
- at least one gripper element; and
- at least one transmission system between said crosspiece element and said telescopic arm.

Conveniently, the biological material is contained in culture plates. The device comprises at least one support for the culture plates.

Advantageously, the device comprises a plurality of temperature sensors and a plurality of heating elements.

Advantageously, said at least one incubator unit comprises at least one first interface for the said at least one input/output unit of the biological material comprising:
- at least one closure element for closing the fluid communication between said incubator unit and the said input/output unit of the biological material;
- at least one duct-like body for the passage of the telescopic arm;
- at least one heating element; and
- at least one temperature sensor.

Preferably, the microscope comprises:
- at least one support table movable along two orthogonal axes;
- at least two micromanipulators;
- two controls for micromanipulators.

Preferably, the incubator arm comprises:
- a main body in fluid communication with the said incubator unit;
- at least one upper observation glass;
- at least two holes for letting said micromanipulators in/out the said main body;
- at least temperature sensors and heating elements for keeping the temperature inside said main body constant or varying it.

Advantageously, each of said holes has a conical shape for allowing a regular outflow of the air from the inside to the outside of the main body.

Conveniently, the main body comprises a hollow space in fluid communication with the inside and the outside of the said incubator arm for allowing the outflow of the pressurized air from said incubator arm.

Preferably, there is further provided a device for adjusting the supporting height of the said incubator arm on said support table.

Further features and advantages of the invention will become more apparent form the detailed description of some preferred but not exclusive embodiments of a device for culturing, controlling and protecting the development of an embryo in vitro according to the present invention.

### BRIEF DESCRIPTION OF THE DRAWINGS

Such a description will be presented hereinafter with reference to the accompanying drawings, provided only for indicating, and thus non-limiting, purposes, wherein:
- figure 1 is a schematic perspective view of the device for culturing, controlling and protecting the development of an embryo in vitro according to the present invention;
- figure 2 is a schematic perspective view of the input/output unit of the biological material;
- figure 3 is a schematic view of the pressurized air circuit according to the present invention;

- figure 4 is a schematic perspective view of the robot of the incubator unit according to the present invention;
- figure 5 is a schematic perspective view of the telescopic arm of the robot according to the present invention;
- figure 6 is a schematic view of the inside of the incubator unit with the telescopic arm of the robot exiting through the second interface according to the present invention;
- figure 7 is a schematic perspective view of the work unit with a microscope according to the present invention;
- figure 8 is a schematic perspective view, partially exploded, of the work unit with microscope according to the present invention;
- figure 9 is a schematic partial sectional view of the incubator arm of the work unit with microscope according to the present invention;
- figure 10 is a schematic view of the lower shell of the incubator arm according to the present invention.

### DETAILED DESCRIPTION OF EMBODIMENTS OF THE INVENTION

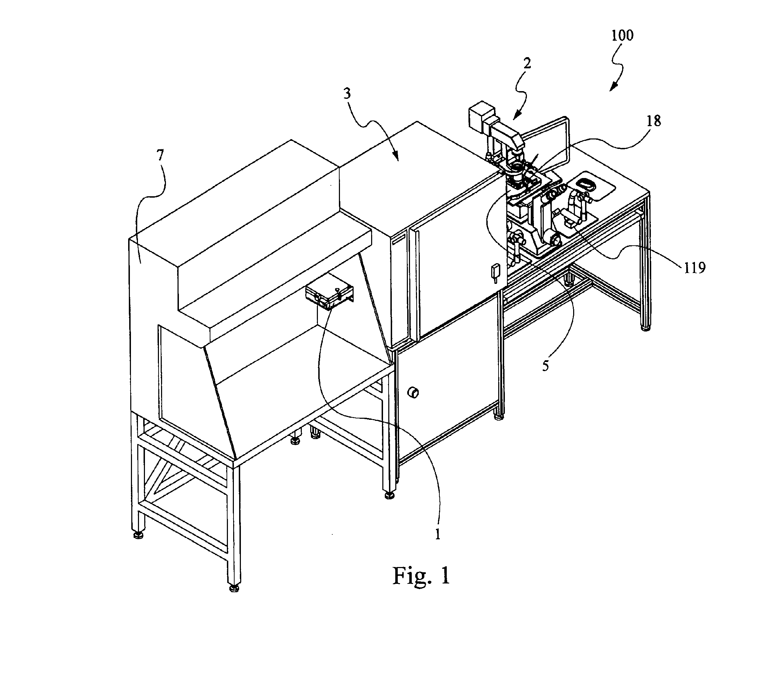

Referring to figures 1 - 10, a device for culturing, controlling and protecting the development of an embryo in vitro according to the present invention is indicated by reference numeral 100.

The device 100, as shown in figure 1, essentially consists of three modular units in communications with one another by means of a handling system and of automated openings which allow the culture plates, containing the biological material, to be transferred from one unit to another without compromising the conditioning of the controlled environment inside the device itself. The units forming the device 100 essentially are an incubator unit 3, a work unit 2 with microscope, and at least one input/output unit 1 of the biological material.

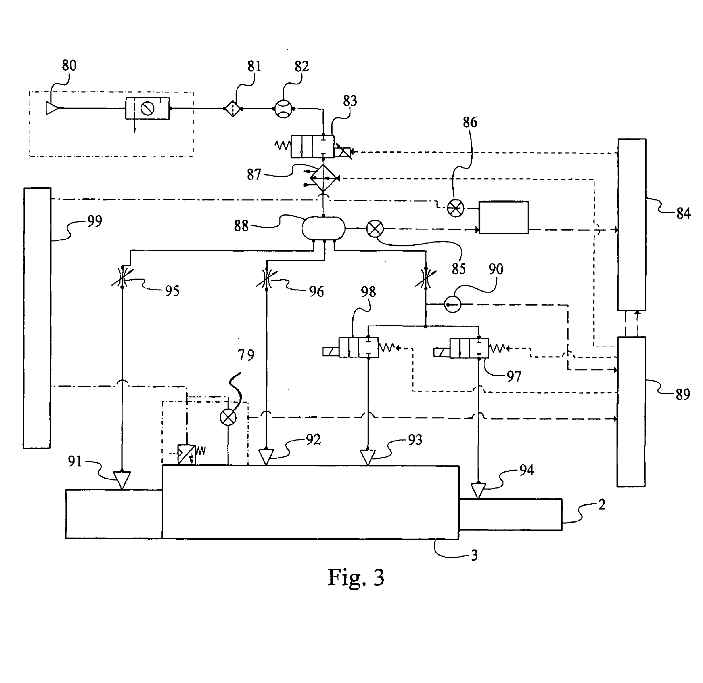

An air over-pressurization system is provided so that sterility can be preserved inside the device. In this way, the over-pressurized air leaves the device 100 in a controlled way, through dedicated openings, hereinafter described, designed so that the access of dust and living organisms into the device 100 is prevented. The air over-pressurization is achieved by means of a circuit, schematically shown in figure 3.

As shown in figure 3, the air which is used is filtered, heated and set to the optimal pressure by means of a control system.

The pressurized air circuit comprises at least one pressurized air source 80, at least one air filter 81, at least one flowmeter 82, at least one flow control valve 83 which allows, when controlled by a dedicated pressure controller 84, the pressure inside the device 100 to be adjusted. The pressure controller 84 is based on three detection sensors 85, 86, 79, of which two sensors 79, 85 are inside the device 100 and one sensor 86 is outside the same and is adapted to detect the air pressure in the laboratory 99 where the device 100 is located.

Downstream the flow control valve 83 a heat exchanger 87 and an accumulator 88 are further provided. The heat exchanger 87, controlled by a main controller 89, connected, among others, with the controller 84, allows the temperature of the air flow entering into the device 100 to be adjusted.

The main controller 89 for detecting the temperature is based on at least one thermal sensor 90.

Downstream the accumulator 88 four inlets are present for letting the pressurized air into the device 100.

In detail, inlet 91 to the input/output unit 1 of the biological material, inlets 92, 93 to the said incubator unit 3 and inlet 94 to the work unit 2 with microscope.

Inlets 91, 92 are controlled by dedicated valves 95, 96 so as to be always active. They are intended for maintaining a minimum overpressure inside the incubator unit 3 and the input/output unit 1 of the biological material.

Inlet 94 is operated by the respective valve 97 when biological material is not present in the unit 2 with microscope. On the contrary, inlet 93 is operated when it is desired to bring the biological material into the unit 2 with microscope by means of the robot 31 (hereinafter described). This change in the air flow is done for forcing the transfer of the atmosphere (air + CO2 + humidity) from the incubator unit 3 to the unit 2 with microscope. This operation is done for two reasons: firstly, for ensuring that the biological material is always immersed in the optimal atmosphere, secondly for reducing the carbon dioxide consumption to the strictly required amount.

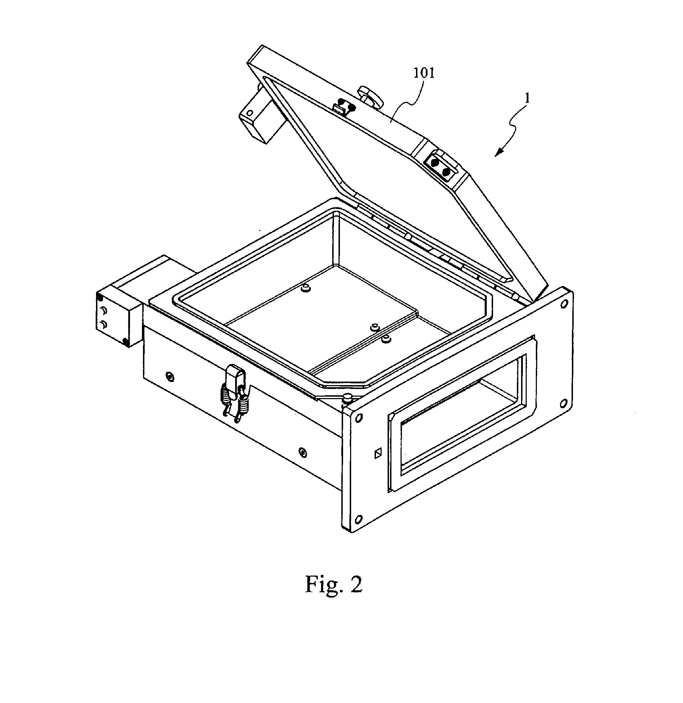

In figure 2 an embodiment of the input/output unit 1 of the biological material is shown in an enlarged view.

The input/output unit 1 of the biological material is in fluid communication with the incubator unit 3. The input/output unit 1 is mainly intended to allow the operator to take from and place into the device 100 biological material. It is provided with a door 101, a heating system and at least one temperature sensor for controlling the temperature.

The door 101 is operated by a solenoid, not show in the figure, and can be opened only at appropriate times.

The input/output unit 1 of the biological material is contained in a laminar flow hood 7. The laminar flow hood 7 is an element known to the person skilled in the art and it is available on the market, therefore it is not further described.

The input/output unit 1 of the biological material communicates with the incubator unit 3 by means of a first interface 12, which is intended for controlling the access of the biological material into the incubator unit 3 and for preserving the air-conditioned environment inside the latter.

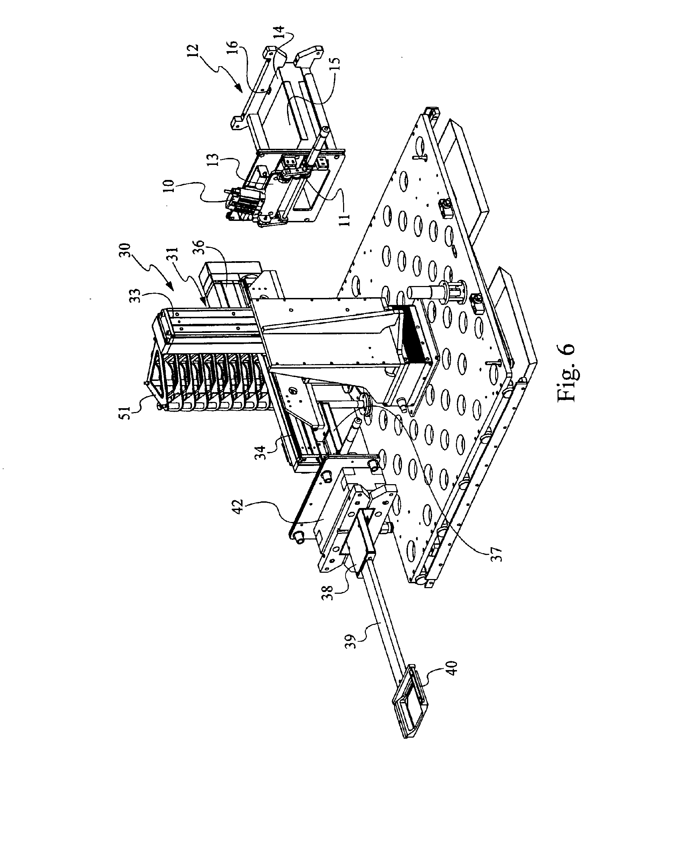

The first interface 12, as better shown in figure 6, comprises at least one duct-like body 14 through which the telescopic arm 37 bringing the biological material can pass, as it will be hereinafter described.

At the inlet of the duct-like body 14, i.e. at the end thereof facing towards the inside of the incubator unit 3, a closure element 13 for closing the fluid communication between said incubator unit 3 and the said input/output unit 1 of the biological material is provided.

In detail, the closure element 13 is represented by a motorized door. The door motor, of electrical type, is connected with a gear train 11 and opens and closes the door. Advantageously, for reducing the opening encumbrance, the door is configured so that as it opens and closes, it remains always parallel to the inlet of the duct-like body 14.

The duct-like body 14 further has at least one temperature sensor 16 and at least one heating element 15, partially wound on the duct-like body 14, for maintaining the desired temperature also in this portion of the device 100.

As shown in figure 6, on the door of the duct-like body 14 an identification code reader, such as a matrix reader 10, can be provided, for reading possible identification codes associated with the culture plates and/or with the biological material.

The biological material required for the in vitro fecundation process is contained in special culture plates 50 made of polymeric material. Such plates, specially designed, allow automatic renewal of the culture ground by exploiting a rocking movement for which the system is designed. However, it is also possible to use conventional plates presently on the market.

The incubator unit 3 represents the core of the device and comprises at least one handling system 30, i.e. a robot 31, preferably having a cylindrical configuration, for carrying the biological material and at least one storing space 51 for the culture plates 50.

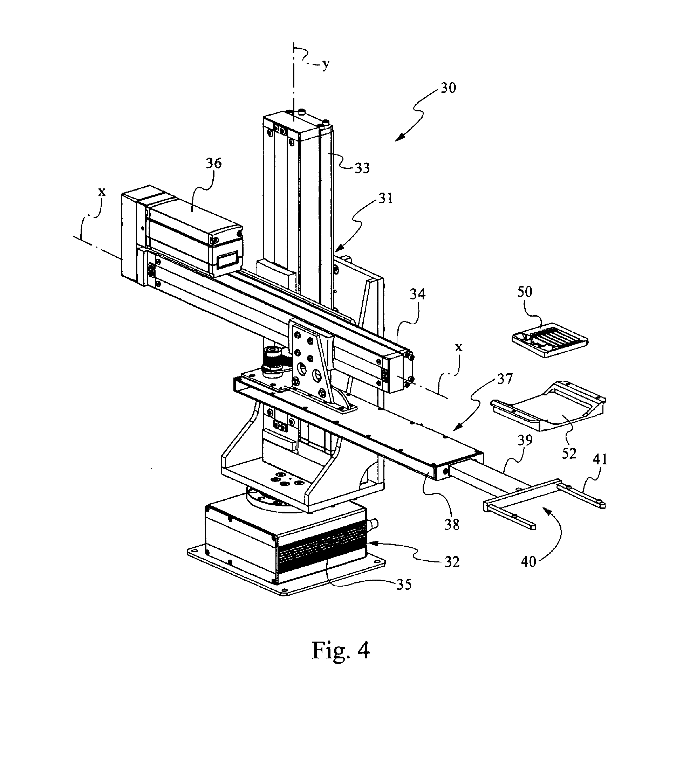

In detail, as better shown in figure 4, the robot 31 has a base body 32, at least one upright element 33, rotatable relative to said base body 32, and at least one crosspiece element 34, translatable along the upright element 33.

Alternatively, the upright element 33 could be translatable relative to said base body 32.

The upright body 33 extends vertically along an axis Y-Y orthogonal to the base body 32 and can rotate about the axis Y-Y relative to the base body 32 thanks to a motor, preferably an electric motor.

The crosspiece element 34 extends orthogonally relative to the upright element 33, along an axis X-X and can translate vertically, i.e. along the axis Y-Y, relative to the upright element 33 thanks to a motor 36, preferably an electric motor.

The robot 31 further comprises a telescopic arm 37, movable along said crosspiece element 34. In other words, the telescopic arm can slide along the axis X-X;

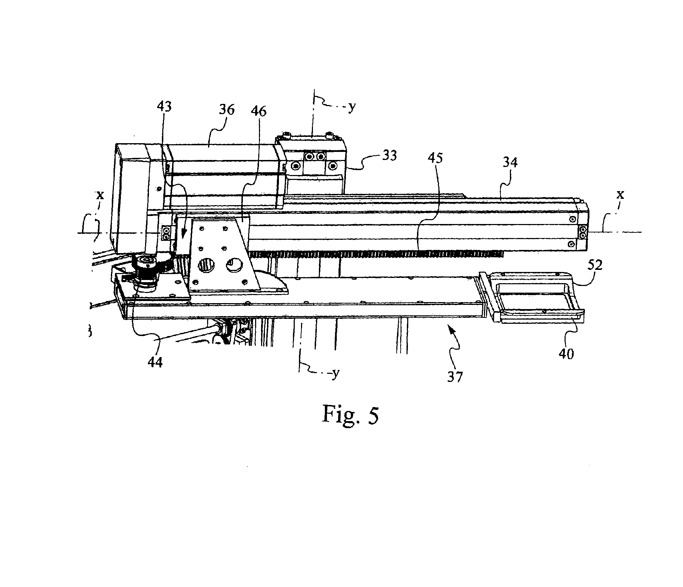

The telescopic arm 37, as shown in figures 4 and 5, has at least two telescopically slidable tubular elements, in detail an outer tubular element 38 and an inner tubular element 39.

The inner tubular element 39 can slide relative to the outer tubular element 38 in a direction substantially parallel to the axis X-X.

The inner tubular element 39 bears, at an end thereof, at least one gripper element 40.

The gripper element 40, in the embodiment shown in figures 4 - 6, is a fork 41 adapted to couple with a special support 52 for the culture plates 50.

At least one transmission system 43 is provided between said crosspiece element 34 and the telescopic arm 37.

In particular, in the embodiment of the figures, in order to reduce the number of actuators present inside the incubator unit 3, a drag transmission system 43, shown in figure 5, has been developed.

The drag transmission system 43 comprises a belt motion transmission system (not visible), a gear train 44, a rack 45 secured to the body of the crosspiece element 34 and a slider 46 steadily connected to the telescopic arm 37, in detail to its outer tubular element 38.

When the telescopic arm 37 is actuated, i.e. during the translation of the latter relative to the crosspiece element 34, the slider 46 moves along the axis X-X. The gear train 44, by engaging with the steady rack 45, determines a rotary motion. The rotary motion is converted into a linear motion by the belt transmission system, so as to make the inner tubular element 39 slide relative to the outer tubular element 38. In this way, when the slider 46, steadily connected to the telescopic arm 37, moves, the gripper member 40 is dragged in the same translation direction of the slider 46.

The motorized arm 37, and thus the gripper member 40, is therefore able to move to any position within the incubator unit 3, for example for taking a culture plate 50 from or putting it back into said storing space 51 for the culture plates 50.

The motorized arm 37 and thus the gripper member 40 can move also outside the incubator unit 3, for example for taking a culture plate 50 from the input/output unit 1 of the biological material and bringing it to the work unit 2 with microscope. As an example, in figure 6 the outer tubular element 39 is shown extracted relative to the inner tubular element 38; therefore, it has passed through the second interface 42 and is thus in a position substantially corresponding to that of the work unit 2 with microscope.

The incubator unit 3 is in communication with the work unit 2 with microscope through the second interface 42.

The second interface 42 is totally similar to the first interface 12, leaving out the extension of the duct-like body 14, and therefore not further described.

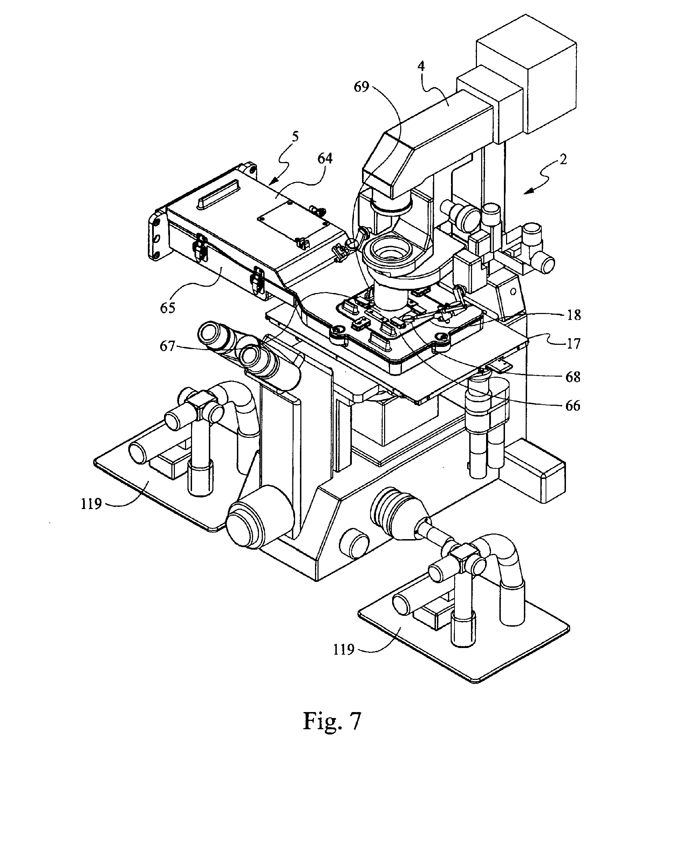

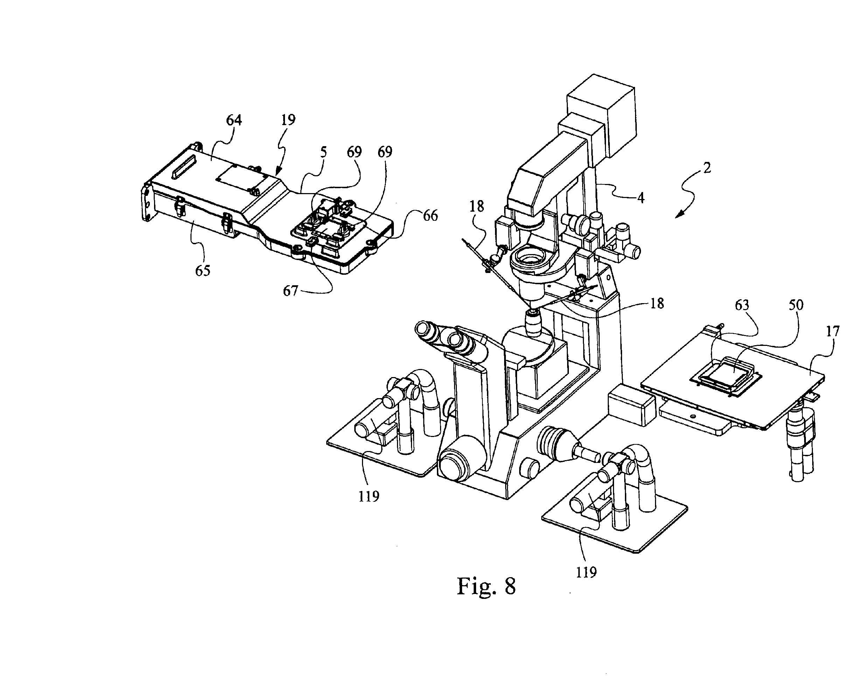

As shown in figures 7 and 8, the work unit 2 with microscope comprises at least one microscope 4 and at least one incubator arm 5 extending from the incubator unit 3 to the microscope 4.

The work unit 2 with microscope has the main task of allowing the monitoring of oocytes and embryos, and the insemination of oocytes through the ICSI technique, keeping the biological material in the ideal environmental conditions.

The microscope 4 of the work unit 2 with microscope is an inverted microscope and further comprises at least one support table 17 movable along two orthogonal, horizontal, axes, and at least two micromanipulators 18.

The inverted microscope 2, the micromanipulation system, i.e. the micromanipulators and the related controls 119, are conventional elements commonly available on the market.

The support table 17 has a heating element 62 for maintaining the desired temperature also in this portion of the device 100. For allowing the observation of the biological material inside the incubator arm 5, a lower observation glass 63 is provided centrally on said support table 17. The lower observation glass 63 is a heating optical glass, electrically powered. The lower observation glass 63, being self-heating, avoids formation of condensate on the surface thereof (which would make the observation of the biological material with the microscope impossible).

The incubator arm 5 is in fluid communication with the said incubator unit 3 and, as mentioned before, extends between the incubator unit 3 and the microscope 4. In detail, the incubator arm 5 is in fluid communication with the incubator unit 3 through the second interface 42.

The incubator arm 5, as better shown in figures 7 - 10, has a main body 19 in fluid communication with the said incubator unit 3.

The main body 19 extends between the incubator unit 3 and the microscope 4 and consists of two removably connectable portions, in detail an upper shell 64 and a lower shell 65, and of a cover 66, which, when the device 100 is assembled, is located at the microscope 4 and is removable from the upper shell 64.

The cover 66 has, in a central position thereof, an upper observation glass 67 and two holes 68 for letting the micromanipulators 18 in/out the main body 19.

The upper observation glass 67, like the lower observation glass 63, allows the biological material inside the incubator arm 5 to be observed and is a heating optical glass, electrically powered. Being self-heating, the upper observation glass 67 avoids the formation of condensate on the surface thereof (which would make the observation of the biological material with the microscope impossible). The system is equipped with a camera for capturing both static and motion images during the observation of the biological material, which are then elaborated by means of a special software.

The two holes 68 for letting the micromanipulators 18 in/out the main body 19 have two removable caps 69.

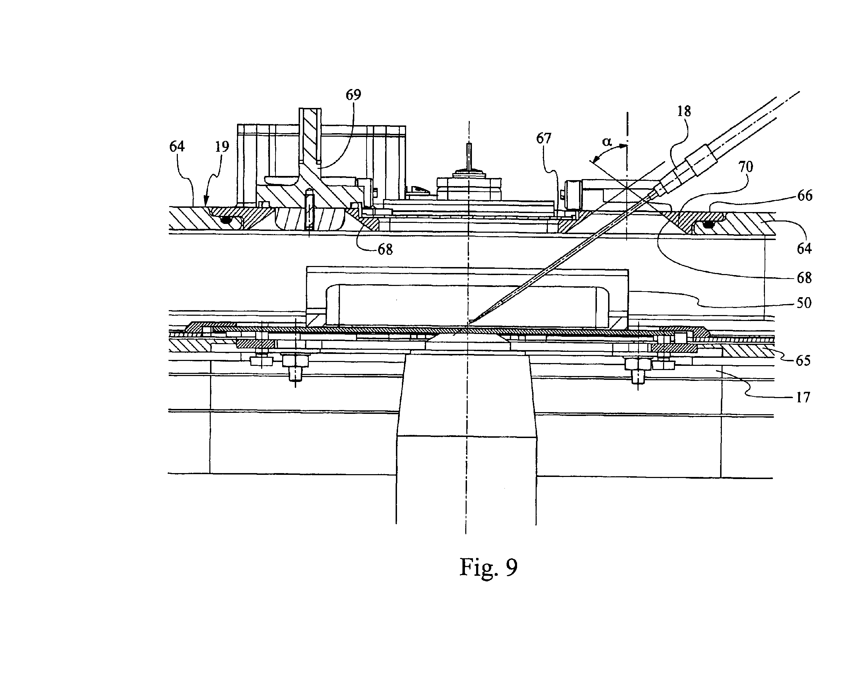

Each of the holes 68 has a conical shape for allowing a regular outflow of the air from the inside to the outside of the main body 19. In other words, as better shown in figure 9, the holes 68 have a conical inner surface 70. In a sectional view the conical surface 70 of the holes 68 forms an angle α relative to the vertical between 5° and 60°, for example equal to about 45°.

The incubator arm 5 further has temperature sensors and heating elements for keeping the temperature inside the said main body (19) constant or varying it.

The main body 19 further has an hollow space 71 created in its lateral walls so as to form a recirculation duct 72 for the pressurized air, which places the inside and the outside of the said incubator arm 5 in fluid communication with each other. In this way the pressurized air can flow out from the incubator arm 5.

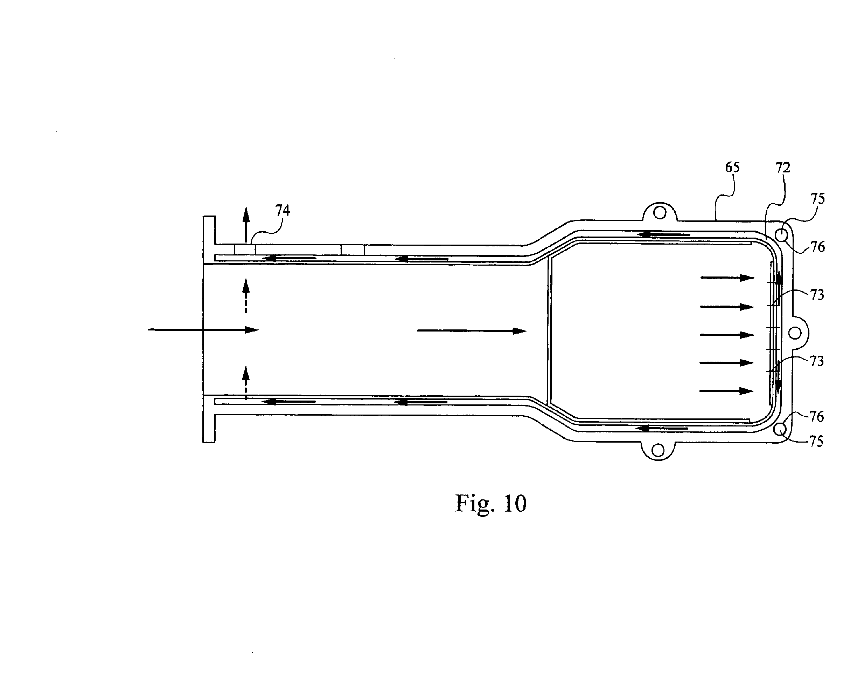

In figure 10 the recirculation duct 72 of the lower shell 65 of the incubator arm 5 and, by means of arrows, the path of the pressurized air coming from the incubator unit 3 are shown.

The recirculation duct 72 runs along the lateral walls of the main body 19 and has air inlet holes 73 provided at the distal end of the incubator arm 5 and an air outlet hole 74 provided at a proximal end of the incubator arm 5.

A spacer device is provided for adjusting the supporting height of the incubator arm 5 relative to the support table 17.

In the embodiment shown in figure 10, the spacer device 75 is formed by two feet 76 provided at the distal end of the main body 19. The two feet 76 are adjustable in height. A different spacer device 75 could be provided.

The present invention has been described with reference to some embodiments thereof. Many modifications can be introduced in the embodiments described in detail, still remaining within the scope of protection of the invention, defined by the following claims.

## Claims

1. Device (100) for culturing, controlling and protecting the development of an embryo in vitro comprising:
- at least one incubator unit (3);
- at least one work unit (2) with microscope; said work unit with microscope comprising at least one microscope (4) and at least one incubator arm (5) extending from said incubator unit (3) to said microscope (4); said incubator arm being in fluid communication with the said incubator unit (3);
- at least one input/output unit (1) of the biological material; said at least one input/output unit (1) of the biological material being in fluid communication with the said incubator unit (3);
- said at least one incubator unit comprising at least one handling system for taking a sample of biological material from said input/output unit (1) and bringing it to a work position at the said microscope and/or vice versa.

2. Device (100) for culturing, controlling and protecting the development of an embryo in vitro according to claim 1, **characterized by** comprising an atmosphere over-pressurization system comprising a pressurized air circuit comprising:
- at least one pressurized air source (80);
- at least one filter (81) for the pressurized air; and
- at least one inlet (91, 92, 93, 94) for letting the pressurized air into said incubator unit (3); said at least one inlet (91, 92 , 93, 94) being controlled by at least one valve (95, 96, 98) and by a controller (89).

3. Device (100) for culturing, controlling and protecting the development of an embryo in vitro according to any one of the previous claims, **characterized in that** said at least one input/output unit (1) of the biological material is contained in a laminar flow hood (7).

4. Device (100) for culturing, controlling and protecting the development of an embryo in vitro according to any one of the previous claims 1 to 3, **characterized in that** said handling system comprises a robot (31) comprising:
- at least one base body (32);
- at least one upright element (33), movable relative to said base body (32); and
- at least one crosspiece element (34), translatable along the upright element (33).

5. Device (100) for culturing, controlling and protecting the development of an embryo in vitro according to claim 4, **characterized in that** said robot (31) comprises:
- a telescopic arm (37), movable along said crosspiece element (34);
- said telescopic arm (37) comprising at least two telescopically slidable tubular elements (38, 39),
- at least one gripper element (40); and
- at least one transmission system between said crosspiece element (34) and said telescopic arm (37).

6. Device (100) for culturing, controlling and protecting the development of an embryo in vitro according to any one of the previous claims, **characterized in that** said biological material is contained in culture plates (50) and the device (100) comprises at least one support for the culture plates.

7. Device (100) for culturing, controlling and protecting the development of an embryo in vitro according to any one of the previous claims, **characterized by** comprising a plurality of temperature sensors and a plurality of heating elements.

8. Device (100) for culturing, controlling and protecting the development of an embryo in vitro according to any one of the previous claims, **characterized in that** said at least one incubator unit (3) comprises at least one first interface (12) for the said at least one input/output unit (1) of the biological material comprising:
- at least one closure element (13) for closing the fluid communication between said incubator unit (3) and the said input/output unit (1) of the biological material;
- at least one duct-like body (14) for the passage of the telescopic arm;
- at least one heating element (15); and
- at least one temperature sensor (16).

9. Device (100) for culturing, controlling and protecting the development of an embryo in vitro according to any one of the previous claims, **characterized in that** said microscope comprises:
- at least one support table (17) movable along two orthogonal axes;
- at least two micromanipulators (18);
- two controls (119) for said micromanipulators (18).

10. Device (100) for culturing, controlling and protecting the development of an embryo in vitro according to any one of the previous claims, **characterized in that** said incubator arm (5) comprises:
- a main body (19) in fluid communication with the said incubator unit (3);
- at least one upper observation glass (20);
- at least two holes (21) for letting said micromanipulators in/out said main body (19);
- at least temperature sensors (21) and heating elements (23) for keeping the temperature inside the said main body (19) constant or varying it.

11. Device (100) for culturing, controlling and protecting the development of an embryo in vitro according to any one of the previous claims, **characterized in that** each of said holes has a conical shape for allowing a regular outflow of the air from the inside to the outside of the main body.

12. Device (100) for culturing, controlling and protecting the development of an embryo in vitro according to any one of the previous claims, **characterized in that** said main body comprises a hollow space in fluid communication with the inside and the outside of the said incubator arm for allowing the pressurized air to flow out from said incubator arm (5).

13. Device (100) for culturing, controlling and protecting the development of an embryo in vitro according to any one of the previous claims, **characterized by** comprising a spacer device for adjusting the supporting height of the said incubator arm (5) on said support table (17).
